# EUROPEAN PATENT APPLICATION

(11) **EP 3 818 972 A2**
(43) Date of publication of application: **12.05.2021**
(21) Application number: 21154417.6
(22) Date of filing: 29.01.2021
(51) Int. Cl.: A61K 8/02, A61K 8/49, A61Q 5/00, A01N 43/40

(54) **PIROCTONE OLAMINE GRANULES FOR USE IN COSMETIC COMPOSITIONS**

(71) Applicant: Clariant International Ltd, 4132 Muttenz (CH)
(72) Inventor: DYBALLA, Michael, 60437 Frankfurt (DE); GROESCHEN, Mathias, 65620 Waldbrunn-Hintermeilingen (DE); HITSCHLER, Lisa, 65779 Kelkheim (Taunus) (DE); KLUG, Peter, 63762 Großostheim (DE)
(74) Representative: Kampen, Daniela

(57) **Abstract**

The present invention relates to the use of piroctone olamine granules for the manufacture of a cosmetic composition, to cosmetic compositions comprising piroctone olamine granules, and to the use of piroctone olamine granules as an anti-dandruff agent or as a preservative.

## Description

The present invention relates to the use of piroctone olamine granules for the manufacture of a cosmetic composition, to cosmetic compositions comprising piroctone olamine granules, and to the use of piroctone olamine granules as an anti-dandruff agent or as a preservative.

With reference to DE 2 234 009 A1 and DE 1 795 270 A1, 1-hydroxy-4-methyl-6-(2,4,4-trimethyl)-pentyl-2(1H)-pyridone, 2-aminoethanol salt, also known as piroctone ethanolamine or piroctone olamine, is an anti-fungal active agent which is effective against the causes of dandruff. It is known to include piroctone olamine in personal care products, such as shampoos. DE 1 795 270 A1 also describes a method of making piroctone olamine. WO2006/081969 describes the use of piroctone olamine as a preservative.

Piroctone olamine exists in the form of crystals which may be added to personal care products. Commercially available piroctone olamine made by processes such as those referred to above, typically has primary crystals with a diameter/length (D/L) ratio of about 1:7 and a median diameter (d₅₀) in the region of about 100 micrometers. The primary crystals are those formed during the crystallization process, but prior to further processing and bulk handling steps. After such further processing and handling in bulk quantities, the crystals may change dimensions. In particular, the primary crystals may fracture and break, such that d₅₀ for such bulk quantities may be smaller than d₅₀ for primary crystals. Such bulk quantities of piroctone olamine crystals may gather together to form clumps, especially during storage. Clumping phenomena may give rise to difficulties when handling and processing the bulk crystals.

It is with this background that the present invention has been devised.

The present invention relates to the use of piroctone olamine granules for the manufacture of a cosmetic composition.

Without wishing to be bound by theory, it is considered that certain particle size ranges, especially certain ranges comprising particle sizes which are larger than the primary crystals, may remain more flowable during storage and be less liable to clumping.

In preferred embodiments, the piroctone olamine granules have a d50 from 0.2 mm to 8 mm, preferably from 0.3 mm to 6 mm, more preferably from 0.4 mm to 2.5 mm, particularly preferably from 0.5 mm to 2 mm.

In preferred embodiments, the piroctone olamine granules have an average D/L ratio of diameter (D) to length (L) of 0.6 or more, preferably of 0.7 or more, particularly preferably of 0.8 or more. In more preferred embodiments, the piroctone olamine granules have an average D/L ratio of diameter (D) to length (L) from 0.6 to 1.0, preferably from 0.7 to 1.0, particularly preferably from 0.8 to 1.0. Without wishing to be bound by theory, it is considered that more "square" or cubic particles may remain more flowable during storage and be less liable to clumping.

In preferred embodiments, the piroctone olamine granules have a d90 of less than or equal to 1.9 mm.

In preferred embodiments, the piroctone olamine granules have a d10 of greater than or equal to 0.4 mm.

In preferred embodiments, the piroctone olamine granules have a bulk density from 400 kg/m³ to 600 kg/m³, preferably from 450 kg/m³ to 550 kg/m³, more preferably from 470 kg/m³ to 530 kg/m³.

In this document, including in all aspects and all embodiments of the present invention, the definitions provided herein apply unless specifically stated otherwise.

In relation to the particle size distribution measures used herein, d₅₀, d50, d(50) or D50, the median, is defined as the diameter where half of the population lies below this value. Similarly, 10 percent of the population lies below the d₁₀, d10, d(10) or D10 diameter and 90 percent of the population lies below the d₉₀, d90, d(90) or D90 diameter. If not stated otherwise, the d₅₀, d₁₀, d₉₀ values are based on a volume distribution.

In preferred embodiments, the piroctone olamine granules are obtained by a process comprising
a) compressing piroctone olamine crystals to form a compactate;
b) milling the compactate to form piroctone olamine granules.
   Advantageously, the process further comprises
c) separating the piroctone olamine granules according to their particle size.

The target particle size range may be determined according to an appropriate metric selected by the skilled person. In one embodiment, the metric is a target d₅₀ range.

The term "compactate" as used herein is well known to a person skilled in the art. The compactate can be in any form. For example, the compactate can be in the form of briquettes, cigars, tablets or "Schülpen". Preferably, the compactate is in the form of briquettes or "Schülpen". Particularly preferably, the compactate is in the form of briquettes. Also particularly preferably, the compactate is in the form of "Schülpen".

Preferably, the compression in a) is performed at a pressure from 25 bar to 200 bar, preferably from 30 bar to 50 bar.

In one embodiment, the pressure applied in compression step a) is sufficient to form a compactate having a density from 920kg/m³ to 1300kg/m³. For completeness, this is the actual density of the compactate, not a bulk density.

The compression in a) may be performed in the absence or presence of additives.

According to one embodiment, pure piroctone olamine crystals, comprising no additives or other materials, are compressed in a). In one embodiment, the compression in a) is performed in the absence of any additives. In one embodiment, the compression in a) is performed in the absence of any plastifiers and lubricants. According to another embodiment, the piroctone olamine crystals are mixed with an additive, such as a plastifier or a lubricant. Suitable additives which may be employed in this instance include polyethylene glycol, stearic acid or ethylene glycol distearate. The compression in a) may be performed in the absence or presence of water. In the compression in a), the amount of water is typically less than 3%, preferably less than 2%, more preferably less than 1%, particularly preferably less than 0.5%.

Preferably, in b) milling takes place in a sieve mill, preferably a rotary sieve mill or an oscillating sieve mill. The mesh of the sieve mill may, for example, have a mesh size of 10 mm or less, preferably of 7 mm or less, more preferably of 4 mm or less, particularly preferably of 2 mm. It is not essential to use a sieve mill, however, and a skilled person would be able to select alternative milling devices.

In one embodiment, in c) separating the piroctone olamine granules comprises using a sieve having a mesh size corresponding to the lower limit of a target particle size range, which yields piroctone olamine fines which have passed through the sieve and piroctone olamine granules in the target particle size range which have not.

In one embodiment, in c) separating the piroctone olamine granules comprises:
c1) a first separation using a first sieve having a mesh size corresponding to the upper limit of a target particle size range, wherein the first separation yields an intermediate product comprising piroctone olamine granules which have passed through the first sieve and a remnant comprising coarse piroctone olamine granules which have not; and
c2) a second separation performed on the intermediate product using a second sieve having a mesh size corresponding to the lower limit of the target particle size range, wherein the second separation yields piroctone olamine fines which have passed through the second sieve and piroctone olamine granules in the target particle size range which have not.

According to another embodiment, in c) separating the piroctone olamine granules comprises using a vibrating sieve or an air jet sieve. According to another embodiment, a first separation is performed with a vibrating sieve or an air jet sieve and/or a second separation is performed with a vibrating sieve or an air jet sieve.

For the event that milling in b) takes place in a mill, such as a sieve mill, that allows granules below a certain size to be generated with a high degree of accuracy, then the first separation may not be necessary, because it may effectively be carried out in the mill. Even then, however, it may still be desirable to perform a first separation, because a small proportion of the granules which are larger than the upper limit of a target particle size range, for example if they are shaped as needles, may pass through a mill, such as a sieve mill.

Advantageously, the process further comprises:
d) recirculating the piroctone olamine fines and adding them to the piroctone olamine crystals to be compressed in a).

Advantageously, for the case in which a first separation has taken place, the process further comprises:
e) milling the remnant;
f) recirculating the milled remnant and adding it to the piroctone olamine crystals to be compressed in a).

Performing one or both of these recirculation steps, while not essential for production of the granules of the final product, is observed to provide a more compact compactate and therefore more compact granules. This is at least partially due to the fact that the recirculated product has been pre-compacted. Recirculation improves the yield of piroctone olamine granules having a target particle size range, not only because recirculation wastes less product, but also because the more compact compactate (and granules) are less friable.

According to the invention, the piroctone olamine granules are used for the manufacture of a cosmetic composition.

In preferred embodiments, from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of the piroctone olamine granules, based on the total weight of the cosmetic composition, are used to manufacture the cosmetic composition.

For example, the cosmetic composition may be selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion.

Preferably, the cosmetic composition is a hair care composition, scalp care composition or skin care composition. More preferably, the cosmetic composition is a hair care or scalp care composition, particularly preferably a hair care and scalp care composition. Also more preferably, the cosmetic composition is a skin care composition.

Preferably, the cosmetic composition is a shampoo composition or hair conditioner composition. More preferably, the cosmetic composition is a shampoo composition, particularly preferably an anti-dandruff shampoo composition. Also more preferably, the cosmetic composition is a hair conditioner composition.

The cosmetic composition preferably comprises one or more further components (F). Preferred further components (F) and preferred amounts of such components are described further below. The further components (F) and amounts thereof described in this document for use in the cosmetic composition of the invention can also be used in the cosmetic composition manufactured according to the invention.

The cosmetic composition can be manufactured by methods known in the art. For example, the cosmetic composition can be manufactured by mixing its ingredients. For example, the piroctone olamine granules can be mixed with the other ingredients of the cosmetic composition.

The invention also relates to a cosmetic composition comprising piroctone olamine granules as described herein. Preferred piroctone olamine granules are described further above.

In preferred embodiments, the cosmetic composition of the invention comprises from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of the piroctone olamine granules, based on the total weight of the cosmetic composition.

For example, the cosmetic composition of the invention may be selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion.

Preferably, the cosmetic composition of the invention is a hair care composition, scalp care composition or skin care composition. More preferably, the cosmetic composition of the invention is a hair care or scalp care composition, particularly preferably a hair care and scalp care composition. Also more preferably, the cosmetic composition of the invention is a skin care composition.

Preferably, the cosmetic composition of the invention is a shampoo composition or hair conditioner composition. More preferably, the cosmetic composition of the invention is a shampoo composition, particularly preferably an anti-dandruff shampoo composition. Also more preferably, the cosmetic composition of the invention is a hair conditioner composition.

In preferred embodiments, the cosmetic composition of the invention is a shampoo composition. The shampoo composition can be in the form of rinse-off products or 'dry shampoo' products, can be opaque or transparent, and can be formulated in a wide variety of product forms, including creams, gels, emulsions, mousses and sprays. Preferably, the shampoo composition is in the form of a rinse-off product. The shampoo composition can, for example, be used on human hair and/or scalp or animal hair, preferably human hair and/or scalp.

In preferred embodiments, the cosmetic composition of the invention is a hair conditioner composition. The hair conditioner composition can be in the form of rinse-off products or leave-on products, can be opaque or transparent, and can be formulated in a wide variety of product forms, including creams, gels, emulsions, mousses and sprays. Preferably, the hair conditioner composition is in the form of a rinse-off product.

The cosmetic composition of the invention preferably comprises one or more further components (F), which can be in an amount of at least 0.01% by weight, preferably at least 0.05% by weight, more preferably at least 0.1% by weight, even more preferably at least 0.5% by weight of the cosmetic composition.

Preferably, the component (F) is selected from the group consisting of acidity regulators, colorants, conditioning agents, emulsifiers, film formers, fragrances, glossers, humectants, lubricants, moisturizers, pigments, preservatives, skin penetration enhancers, stabilizers, surfactants, thickeners, and viscosity modifiers. More preferably, the component (F) is selected from the group consisting of acidity regulators, glossers, lubricants, and surfactants.

Suitable lubricants are, for example, fatty alcohol components having 6 to 18 carbon atoms.

The surfactants may, for example, be selected from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) or cetylpyridinium chloride.

As cationic components, a variety of cationic polymers are suitable, including quaternized cellulose ethers, copolymers of vinylpyrrolidone, acrylic polymers, including homopolymers or copolymers of dimethyldiallylammonium chloride or acrylamide. Also suitable are various types of homo- or copolymers derived from acrylic or methacrylic acid, acrylamide, methylacrylamide, diacetone-acrylamide.

In at least one embodiment, the component (F) is a glosser. Typical glossers are silicones. Suitable as silicones are volatile or nonvolatile non-ionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, or mixtures thereof.

The cosmetic composition of the invention may contain from 0.05 to 5%, preferably 0.5 to 5% by weight of at least one oil component. Typical oils are organic oils, which often are esters. The oil component may comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil.

The cosmetic composition of the invention may contain from 0.05 to 5%, preferably 0.5 to 5% by weight of at least one emulsifier. Preferred emulsifiers are, for example, sorbitan esters.

The cosmetic composition of the invention can contain from 0.1 to 10% by weight, preferably from 0.2 to 5% by weight, more preferably from 0.2 to 3% by weight, also more preferably from 0.5 to 5% by weight of at least one rheology modifying agent, in particular a gelling and thickening agent. Examples are cellulosic thickeners, for example, hydroxyethylcellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol®. Another class of (meth)acylic acid polymers are alkali-swellable emulsion (ASE) polymers. ASE polymers include, for example, Aculyn® 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodynamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn® 22 and Aculyn® 28 copolymers from Dow and Aqua SF 1® copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a C8-C30 alkyl or, as therein more particularly described, a hydrocarbyl monoether of polyethylene glycol, (3) a C1-C4 alkyl acrylate or methacrylate, and, optionally, (4) a small amount of a polyethylenically unsaturated monomer.

The cosmetic composition of the invention (in particular hair conditioner composition) can also comprise as component (F) a fatty compound. The fatty compound may be included in the cosmetic composition at a level of from 0.1 to 20 % by weight, preferably from 1.0 to 10 % by weight. The fatty compound is selected from the group consisting of fatty alcohols (e.g. cetyl alcohol, stearyl alcohol or cetearyl alcohol), fatty acids, fatty alcohol derivatives, fatty acid derivatives, or mixtures thereof.

It is understood that the components disclosed can in some instances fall into more than one classification, e.g., some fatty alcohol derivatives can also be classified as fatty acid derivatives. However, a given classification, is not intended to be a limitation on that particular compound but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, or mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, or mixtures thereof. Examples of fatty alcohol derivatives and fatty acid derivatives include methyl stearyl ether, polyoxyethylene ethers of behenyl alcohol, ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, or mixtures thereof.

The cosmetic composition of the invention may comprise an aqueous carrier. The level and species of the aqueous carrier are selected according to the compatibility with other components and other desired characteristic of the cosmetic composition. The aqueous carrier may, for example, be water or water solutions of lower alkyl alcohols or polyhydric alcohols. The lower alkyl alcohols may, for example, be monohydric alcohols having 1 to 6 carbons, often ethanol and/or isopropanol. The polyhydric alcohols may, for example, be propylene glycol, hexylene glycol, glycerin, and/or propane diol. Preferably, the aqueous carrier is substantially water. Deionized water is preferably used. Water from natural sources, including minerals can also be used, depending on the desired characteristic of the composition. Generally, the cosmetic composition of the invention can comprise up to 80 %, often even up to 95 % by weight of water.

The cosmetic composition of the invention may also include as a further component (F) other components being suitable for rendering the compositions more cosmetically or aesthetically acceptable or to provide them with additional usage benefits. Such other components can generally be used individually at levels of from 0.001 % to 5 % by weight. A wide variety of further components (F) can be formulated into the cosmetic composition of the invention. These include conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers, such as amphoteric fixative polymers, cationic fixative polymers, anionic fixative polymers, non-ionic fixative polymers, silicone grafted copolymers; preservatives, such as benzyl alcohol, methyl paraben, propyl paraben, imidazolidinyl urea; pH adjusting agents, such as citric acid, sodium citrate, succinic acid, phosphoric acid, sodium hydroxide, sodium carbonate; salts, in general, such as potassium acetate or sodium chloride; coloring agents; hair oxidizing (bleaching) agents, such as hydrogen peroxide, perborate or persulfate salts; hair reducing agents such as thioglycolates; perfumes; and sequestering agents, such as disodium ethylene-diamine tetraacetate.

Preferably, salt is present at levels from 0.1 to 1 wt.-% of the total cosmetic composition to adjust the product viscosity. Preferably, NaOH is present at levels from 0.1 to 1 wt.-% of the total cosmetic composition to adjust the pH of the formulation.

The cosmetic composition of the invention may contain as a further component (F) a polysorbate for adjusting rheology, for example, polysorbate-20, polysorbate-21, polysorbate-40, polysorbate-60, or mixtures thereof. The polysorbate can be contained in the cosmetic composition in amounts up to 5% (e.g. 0.1 to 5%) by weight.

The cosmetic composition of the invention can also contain as a further component (F) a polypropylene glycol. Preferred polypropylene glycols are those having a weight average molecular weight of from 200 to 100000 g/mol. The polypropylene glycol may be either water-soluble, water-insoluble, or may have a limited solubility in water, depending upon the degree of polymerization and whether other moieties are attached thereto. The desired solubility of the polypropylene glycol in water will depend in large part upon the form of the composition (e.g., leave-on composition, rinse-off composition). The polypropylene glycol can be included in the cosmetic composition of the invention at a level of up to 10% by weight.

For example, in a rinse-off composition, it is preferred that the polypropylene glycol has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the cosmetic composition of the invention at a level of up to 10% by weight.

The cosmetic composition of the invention can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, or glyceryl ester oils; poly [alpha]-olefin oils; and mixtures thereof. Preferred low melting point oils are selected from the group consisting of ester oils such as pentaerythritol ester oils, trimethylol ester oils, citrate ester oils, or glyceryl ester oils; poly [alpha]-olefin oils; and mixtures thereof. Particularly useful pentaerythritol ester oils and trimethylol ester oils are pentaerythritol tetraisostearate, pentaerythritol tetraoleate, trimethylolpropane triisostearate, trimethylolpropane trioleate, or mixtures thereof. Particularly useful glyceryl esters are triisostearin, triolein or trilinolein.

The cosmetic composition of the invention can also contain, as a further component (F), a cationic polymer. Cationic polymers may be present in the cosmetic composition of the invention for further enhancing deposition performance.

Suitable cationic polymers may be homopolymers which are cationically substituted or may be formed from two or more types of monomers. The weight average (Mw) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups.

Other suitable spacers include vinyl esters, vinyl alcohol, maleic anhydride, propylene glycol and ethylene glycol. The cationic amines can be primary, secondary or tertiary amines, depending upon the particular species and the pH of the composition. In general, secondary and tertiary amines, especially tertiary, are preferred. Amine substituted vinyl monomers and amines can be polymerized in the amine form and then converted to ammonium by quaternization. The cationic polymers can comprise mixtures of monomer units derived from amine- and/or quaternary ammonium-substituted monomer and/or compatible spacer monomers.

Suitable cationic polymers include, for example cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of amino-alkyl esters of homo- and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in the cosmetic composition of the invention include monomers of the formula: A-O-[R-N⁺(R1)(R2)(R3)X⁻], wherein: A is an anhydroglucose residual group, such as a starch or cellulose anhydroglucose residual. R is an alkylene, oxyalkylene, polyoxyalkylene, or hydroxyalkylene group, or combination thereof. R1, R2 and R3 independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R1, R2 and R3) is preferably about 20 or less, and X⁻ is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17, JAGUAR C16, JAGUAR CHT and JAGUAR C162.

Mixtures of any of the above cationic polymers may be used. Cationic polymer may be present in the cosmetic composition of the invention at levels of from 0.01 to 5 wt.-%, preferably from 0.05 to 1 wt.-%, more preferably from 0.08 to 0.5 wt.-% by total weight of cationic polymer based on the total weight of the cosmetic composition.

In at least one embodiment, the cationic polymers have a number average molecular weight of at least about 5000 g/mol, typically from 10000 g/mol to 10 million g/mol and are selected from the group consisting of copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar® from Solvay.

In at least one embodiment, the cosmetic composition of the invention comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the cosmetic composition of the invention comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the cosmetic composition of the invention comprises an anionic surfactant. In at least one embodiment, the anionic surfactant is selected from the group consisting of (C10-C20)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in the cosmetic composition of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, where n is from 1 to 3; more preferably sodium lauryl ether sulphate(n)EO, where n is from 1 to 3; most preferably sodium lauryl ether sulphate(n)EO, where n=1. Preferably, the level of alkyl ether sulphate is from 0.5 wt.-% to 25 wt.-% of the total composition, more preferably from 3 wt.-% to 18 wt.-%, most preferably from 6 wt.-% to 15 wt.-% of the total composition.

The total amount of anionic surfactant in the cosmetic composition of the invention may range from 0.5 wt.-% to 45 wt.-%, more preferably from 1.5 wt.-% to 20 wt.-%.

The cosmetic composition of the invention may comprise fatty acyl isethionate, if present, preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 wt.-%. Preferably, greater than 20 wt.-% and less than 45 wt.-%, more preferably greater than 25 wt.-% and less than 45 wt.-% of the fatty acyl isethionate are of chain length greater than or equal to C16; and greater than 50 wt.-%, preferably greater than 60 wt.-% of the free fatty acid/soap is of chain length C16 to C20. In addition, the product may contain isethionate salts, which are present typically at levels less than 5 wt.-%, and traces (less than 2 wt.-%) of other impurities.

Preferably, a mixture of aliphatic fatty acids is used for the preparation of commercial fatty acyl isethionate surfactants. The resulting fatty acyl isethionate surfactants (e.g., resulting from the reaction of alkali metal isethionate and aliphatic fatty acid) preferably should have more than 20 wt.-%, preferably more than 25 wt.-%, but no more than 45 wt.-%, preferably 35 wt.-% (on basis of fatty acyl isethionate reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the cosmetic composition of the invention comprises an acylglycinate surfactant. In at least one embodiment, the acylglycinate surfactant conforms to the formula (Y): 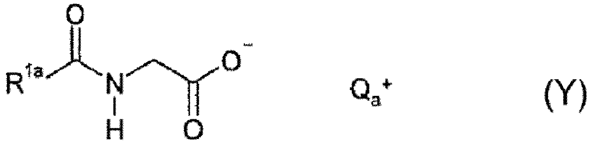 wherein
R^{1a} is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22, particularly preferably 12 to 18 carbon atoms, and
Qₐ⁺ is a cation.

In at least one embodiment, Qₐ⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C12 alkyl or C14 alkyl. In at least one embodiment, the acylglycinate surfactant is selected from those conforming to formula (Y), wherein R is C16 alkyl or C18 alkyl.

In at least one embodiment, the cosmetic composition comprises from 0.01 wt.-% to 30 wt.-%, or from 1 wt.-% to 25 wt.-%, preferably from 5 wt.-% to 20 wt.-%, more preferably from 12 wt.-% to 18 wt.-% anionic surfactant.

In at least one embodiment, the cosmetic composition of the invention comprises a glutamate surfactant corresponding to formula (Z) or a salt thereof: 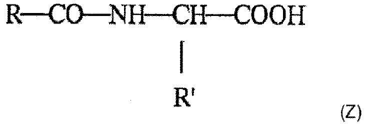 wherein R' is HOOC-CH2-CH2- or M⁺⁻OOC-CH2-CH2- wherein M⁺ is a cation; and wherein R is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C12 alkyl or C14 alkyl. In at least one embodiment, the glutamate surfactant is selected from those conforming to formula (Z), wherein R is C16 alkyl or C18 alkyl.

In at least one embodiment, the cosmetic composition of the invention comprises a non-ionic surfactant. Non-ionic surfactants may be present in the range 0 to 5 wt.-%. Non-ionic surfactants that can be included in the cosmetic composition of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

R-(OCH₂CH₂)nOH,

where R is an alkyl chain of C12 to C15, and n is 5 to 9. Other suitable non-ionic surfactants include mono- or di-alkyl alkanolamides. Examples include coco mono- or diethanolamide and coco mono-isopropanolamide.

Further non-ionic surfactants which can be included in the cosmetic composition of the invention are alkyl polyglycosides (APGs). Typically, APG is one which comprises an alkyl group connected (optionally via a bridging group) to a block of one or more glycosyl groups. Preferred APGs are defined by the following formula:

RO-(G)n

wherein R is a branched or straight chain alkyl group which may be saturated or unsaturated, and G is a saccharide group. R may represent a mean alkyl chain length of from about C5 to about C20. Preferably R represents a mean alkyl chain length of from about C9 to about C12. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies between about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived non-ionic surfactants which can be included in the cosmetic composition of the invention include fatty (e.g. C10-C18) N-alkyl (C1-C6) polyhydroxy fatty acid amides, such as C12-C18 N-methyl glucamides, as described for example in WO9206154 and US5194639, and N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- and diesters of addition products of 1 to 30 mol of ethylene oxide onto glycerol, addition products of 5 to 60 mol of ethylene oxide onto castor oil or onto hydrogenated castor oil, fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C8- to C22-fatty acids and a degree of ethoxylation of from 4 to 20, polyglyceryl fatty acid esters, in particular of one, two or more C8- to C22-fatty acids and polyglycerol having preferably 2 to 20 glyceryl units, alkyl glucosides, alkyl oligoglucosides and alkyl polyglucosides having C8 to C22-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics®), fatty acid alkylol amides, (fatty acid amide polyethylene glycols), N-alkyl-, N-alkoxypoly-hydroxy-fatty acid amide, sucrose esters, sorbitol esters, polyglycol ethers, and mixtures thereof.

In at least one embodiment, the cosmetic composition of the invention comprises a fatty N-methyl-N-glucamide surfactant. In at least one embodiment, the fatty N-methyl-N-glucamide surfactant conforms to the formula (X): 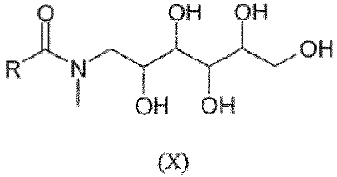 wherein
R is a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1 butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2 methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3 methyl-2-pentyl, 4-methyl-2-pentyl, 2 methyl-3-pentyl, 3-methyl-3-pentyl, 2,2 dimethyl-1-butyl, 2,3-dimethyl-1-butyl, 3,3 dimethyl-1-butyl, 2-ethyl-1-butyl, 2,3-dimethyl-2-butyl, 3,3-dimethyl-2-butyl, 1 heptyl, 1-octyl, 1-nonyl, 1-decyl, 1 undecyl, 1-dodecyl, 1-tetradecyl, 1-hexadecyl and 1-octadecyl. Suitable fatty N methyl-N-glucamide surfactants are described in WO2013/178700 and EP 0 550 637, which are incorporated herein by reference. In at least one embodiment, the N-methyl-N-glucamide surfactant is selected from those conforming to formula (X), wherein R is C12 alkyl or C14 alkyl. In at least one embodiment, the N-methyl-N-glucamide surfactant is selected from those conforming to formula (X), wherein R is C16 alkyl or C18 alkyl.

In at least one embodiment, the cosmetic composition of the invention comprises from 1 wt.-% to 20 wt.-%, more preferably from 2 wt.-% to 10 wt.-%, even more preferably from 3 wt.-% to 7 wt.-% non-ionic surfactant.

Amphoteric or zwitterionic surfactant can be included in the cosmetic composition of the invention in an amount ranging from 0.5 wt.-% to about 8 wt.-%, preferably from 1 wt.-% to 4 wt.-% of the total composition.

In at least one embodiment, the amphoteric surfactants are selected from the group consisting of N-(C₁₂-C₁₈)-alkyl-beta-aminopropionates and N-(C₁₂-C₁₈)-alkyl-beta-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine; (C₁₂-C₁₈)-alkyl-dimethyl-sulfopropylbetaine; amphosurfactants based on imidazoline (trade name: Miranol®, Steinapon®), preferably the sodium salt of 1-(beta-carboxymethyloxyethyl)-1-(carboxymethyl)-2-laurylimidazolinium; amine oxide, e.g., (C₁₂-C₁₈)-alkyl-dimethylamine oxide, fatty acid amidoalkyldimethylamine oxide, and mixtures thereof.

In at least one embodiment, the cosmetic composition of the invention comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C8- to C18-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C8- to C18-alkyldimethylammonium acetates, the C8- to C18 alkyldimethylcarbonylmethylammonium salts, and the C8- to C18-fatty acid alkylamidobetaines, and mixtures thereof. Optionally, the C8- to C18-fatty acid alkylamidobetaine is selected from coconut fatty acid amidopropylbetaine, N-coconut fatty acid amidoethyl-N-[2-(carboxymethoxy)ethyl]glycerol (CTFA name: cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the cosmetic composition of the invention comprises from 0.5 wt.-% to 20 wt.-%, preferably from 1 wt.-% to 10 wt.-% amphoteric surfactant.

In at least one embodiment, the cosmetic composition of the invention comprises a surfactant system. In at least one embodiment, the surfactant system comprises at least one surfactant selected from the group consisting of lauryl sulfate, laureth sulfate, cocamidopropyl betaine, sodium cocoylglutamate, lauroamphoacetate, and mixtures thereof. In at least one embodiment, the surfactant system comprises sodium laureth sulfate, sodium lauryl sulfate, and optionally cocamidopropyl betaine. In at least one embodiment, the surfactant system comprises sodium laureth sulfate, potassium cocoylglutamate, and cocamidopropyl betaine.

In at least one embodiment, the cosmetic composition of the invention contains as a further component a silicone compound. The cosmetic composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, non-ionic surfactants, cationic surfactants, and mixtures thereof.

In at least one embodiment, the cosmetic composition of the invention contains silicone conditioning agents. Preferably, these are emulsified droplets of a silicone conditioning agent. These are for enhancing conditioning performance.

Suitable silicones include polydiorganosiloxanes, in particular polydimethylsiloxanes, which have the CTFA designation dimethicone. Also suitable for use in the cosmetic composition of the invention are polydimethyl siloxanes having hydroxyl end groups, which have the CTFA designation dimethiconol. Also suitable for use in the cosmetic composition of the invention are silicone gums having a slight degree of cross-linking, as are described for example in WO 96/31188. The viscosity of the emulsified silicone itself (not the emulsion or the final composition) is typically at least 10,000 cSt at 25°C. The viscosity of the silicone itself is preferably at least 60,000 cSt, most preferably at least 500,000 cSt, ideally at least 1,000,000 cSt. Preferably, the viscosity does not exceed 1x10⁹ cSt for ease of formulation. Emulsified silicones for use in the cosmetic composition of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in the cosmetic composition of the invention are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone". Specific examples of amino functional silicones suitable for use in the cosmetic composition of the invention are the aminosilicone oils DC2-8220, DC2-8166 and DC2-8566 (all ex Dow Corning). Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactants. Pre-formed emulsions of amino functional silicones are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning).

Combination of amino and non-amino functional silicones may also be used.

The total amount of silicone is preferably from 0.01 wt.-% to 10 wt.-% of the total composition, more preferably from 0.1 wt.-% to 5 wt.-%, most preferably from 0.5 wt.-% to 3 wt.-%.

In at least one embodiment, the cosmetic composition of the invention comprises a preservative or preservative system. Examples of suitable preservatives include benzyl alcohol, phenoxyethanol, parabens, benzoic acid/sodium benzoate, sorbic acid/potassium sorbate, and other organic acids used to provide antimicrobial protection. In at least one embodiment, the cosmetic composition comprises from 0.01 to 5 wt.-%, particularly preferably from 0.05 to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethylsarcosinate, lauroylsarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 3,4,4'-trichlorocarbanilide (Triclocarban), diaminoalkylamide, L-lysine hexadecylamide, heavy metal citrate salts, zinc salts, zinc phenol sulfate, farnesol, naftifine, terbinafine, selenium disulfide, methylchloroisothiazolinone, methylisothiazolinone, methyldibromo glutaronitrile, AgCI, chloroxylenol, sodium salts of diethylhexylsulfosuccinate, sodiumbenzoate, phenoxyethanol, benzyl alcohol, phenoxyisopropanol, paraben, such as butyl-, ethyl-, methyl- und propylparaben, and their salts, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition of the invention is substantially free of parabens.

The cosmetic composition of the invention may also comprise a dispersed, non-volatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C8-C22 carboxylic acids. Preferred materials include cocoa butter, palm stearin, sunflower oil, soybean oil and coconut oil.

The oily or fatty material may be present at a level of from 0.05 to 10 wt.-%, preferably from 0.2 to 5 wt.-%, more preferably from 0.5 to 3 wt.-%, based on the total weight of the cosmetic composition.

In a particularly preferred embodiment, the composition of the invention is a shampoo composition.

In at least one embodiment, the shampoo composition comprises from 1 to 99%, preferably from 5 to 95%, more preferably from 10 to 90% by weight of the total composition of water, and from 0.1 to 99%, preferably from 1 to 95%, more preferably from 5 to 90%, often from 5 to 25% by weight of the total composition of a cleansing surfactant. Suitable cleansing surfactants are generally anionic, amphoteric, betaine, or zwitterionic surfactants. For example, the anionic surfactants are alkyl ether or alkyl ether sulfates, such as sodium lauryl sulfate, or other compounds described above.

In at least one embodiment, the shampoo composition comprises one or more further cosmetically acceptable components (F), which can be present in an amount of at least 0.5% by weight, or from 0.5 to 20% by weight, by total weight of the shampoo composition. Preferably, the component (F) is selected from the group consisting of cleansing ingredients, acidity regulators, colorants, conditioning agents, emulsifiers, film formers, fragrances, glossers, humectants, lubricants, moisturizers, pigments, preservatives, hair penetration enhancers, scalp actives, stabilizers, surfactants, thickeners, viscosity modifiers, and combinations thereof. More preferably, the component (F) is selected from the group consisting of surfactants, viscosity-modifying polymers and conditioning ingredients. In at least one embodiment, the shampoo composition comprises from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of the piroctone olamine granules, and at least 0.5% by weight of one or more further components (F) selected from the group consisting of surfactants, polymers, conditioning agents, actives, acidity regulators, lubricants, moisturizers, oils, preservatives, sequestrants, strengtheners, sun protectors, and combinations thereof.

In at least one embodiment, the shampoo composition comprises further cosmetically acceptable components (F) being cleansing ingredients. In at least one embodiment, the shampoo composition comprises from 0.05 to 20% by weight of cleansing ingredients, based on the total weight of the shampoo composition. In at least one embodiment of the shampoo composition, the level of cleansing ingredient is from 1% to 20% by weight, preferably from 5% to 18%, more preferably from 8% to 16%, based on the total weight of the shampoo composition. In at least one embodiment, the cleansing ingredient is selected from the group consisting of non-polymeric surfactants, saponins, polymeric surfactants, and combinations thereof. Preferably, the cleansing ingredient comprises or consists of surfactants.

In at least one embodiment, the shampoo composition comprises from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of the piroctone olamine granules, and at least 0.5 % by weight of surfactants, preferably cleansing anionic or non-ionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the shampoo composition is silicone-free. In at least one embodiment, the shampoo composition is sulfate-free. In at least one embodiment, the shampoo composition is silicone-free and sulfate-free.

In at least one embodiment, the shampoo composition comprises, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the piroctone olamine granules;
(ii) from 5 wt.-% to 20 wt.-% of one or more anionic surfactants;
(iii) at least 50 wt.-% water; and
(iv) at least one further cosmetically acceptable component (F) selected from the group consisting of silicone, cationic polymer, rheology modifiying agent, and an amphoteric or zwitterionic surfactant.

In at least one embodiment, the shampoo composition comprises, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the piroctone olamine granules;
(ii) from 5 wt.-% to 20 wt.-% of one or more anionic surfactants;
(iii) at least 50 wt.-% water;
(iv) at least one further component selected from the group consisting of silicone, cationic polymer, rheology modifiying agent, and an amphoteric or zwitterionic surfactant; and
(v) at least one further cosmetically acceptable component (F).

In at least one embodiment, the shampoo composition consists of, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the piroctone olamine granules;
(ii) from 5 wt.-% to 20 wt.-% of one or more anionic surfactants;
(iii) at least 50 wt.-% water;
(iv) at least one further component selected from the group consisting of silicone, cationic polymer, rheology modifiying agent, and an amphoteric or zwitterionic surfactant; and
(v) at least one further cosmetically acceptable component (F) selected from the group consisting of conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers such as amphoteric fixative polymers, cationic fixative polymers, anionic fixative polymers, non-ionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents, such as the thioglycolates; perfumes; and sequestering agents, such as disodium ethylene-diamine tetraacetate.

The cosmetic composition of the invention can be manufactured by methods known in the art. For example, the cosmetic composition of the invention can be manufactured by mixing its ingredients. For example, the piroctone olamine granules can be mixed with the other ingredients of the cosmetic composition of the invention.

The invention also relates to the use of piroctone olamine granules as described herein as an anti-dandruff agent or as a preservative. In preferred embodiments, the invention relates to the use of piroctone olamine granules as described herein as an anti-dandruff agent. In preferred embodiments, the invention relates to the use of piroctone olamine granules as described herein as a preservative.

Particularly preferably, the piroctone olamine granules are incorporated into a cosmetic composition. Preferably, the cosmetic composition is a shampoo composition or hair conditioner composition. More preferably, the cosmetic composition is a shampoo composition, particularly preferably an anti-dandruff shampoo composition. Also more preferably, the cosmetic composition of the invention is a hair conditioner composition.

Preferably, the dandruff is caused by dandruff-causing organisms, more preferably Malassezia species, even more preferably Malassezia furfur and/or Malassezia globosa, particularly preferably Malassezia furfur, also particularly preferably Malassezia globosa.

The invention also relates to a method of treating hair and/or scalp, comprising:
a) applying a shampoo composition and/or hair conditioner composition (preferably shampoo composition) as described herein onto wet hair and/or scalp and then
b) removing the shampoo composition and/or hair conditioner composition (preferably shampoo composition) from the hair and/or scalp.

All percentages are by weight (w/w) of the total composition. All ratios are weight ratios. "wt.%" means percentage by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. If not stated otherwise, all measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Embodiments and aspects described herein may comprise or be combinable with elements, features or components of other embodiments and/or aspects despite not being expressly exemplified in combination, unless an incompatibility is stated. "Molecular weight" or "M.Wt." or "MW' and grammatical equivalents mean the number average molecular weight, if not stated otherwise.

The invention will now be further described with reference to the accompanying drawings, in which:
Figure 1 illustrates the volumetric distribution density of particles, q (left hand y-axis) and the volumetric cumulative distribution Q(r) (right hand y-axis) versus diameter (x-axis) of crystals of piroctone olamine which have not been subjected to the process according to the invention.
Figure 2 illustrates the volumetric distribution density of particles, q (left hand y-axis) and the volumetric cumulative distribution Q(r) (right hand y-axis) versus diameter (x-axis) of granules of piroctone olamine which have been subjected to the process according to the invention.
Figure 3 illustrates a microscope image of some piroctone olamine granules according to the invention. The image is divided into 4 parts using a reticle scale in preparation for making width and length measurements in the fashion described below.

### Particle Size Distribution (PSD) Measurement Method for Piroctone Olamine Crystals

For measuring the PSD of the piroctone olamine crystals (the starting material), a Horiba LA-950 particle size analyzer was used for measuring the diameter, the volumetric distribution density and the volumetric cumulative distribuition of the crystals. The analyzer uses a laser diffraction method (ISO 13320:2009, Fraunhofer Diffraction Method) to measure the distribution and is based on the direct proportionality of the intensity of light scattered by a particle to the diameter. Furthermore, the scattering angle is inversely proportional to the diameter and vice versa.

In preparation for the analysis, the required amount of crystals is placed on a sieve with a mesh size of 1 mm. The crystals are sieved with an amplitude of 1.5 mm for 3 minutes.

The required amount of sieved crystals was added to the gutter of the dry dispersion unit.

Three measurements were made in the HORIBA LA-950 particle size analyzer with the following parameters:
- Gutter starting value 85 -110 (unit-less), automatic control
- Dispersing pressure 0.3 MPa

The three measurements are combined with the software to form an averaged measurement. For analysis the focus is on d₁₀, d₅₀ and d₉₀ volume fractions.

### Particle Size Distribution Measurement Method of Piroctone Olamine Granules

For piroctone olamine granules, which have been compressed according to the invention, a Retsch Sieve Machine "AS200 Control" was used for measuring the diameter, the volumetric distribution density and the volumetric cumulative distribution of the granules. This is referred to herein as a sieve analysis.

In preparation for the sieve analysis, sieves are stacked one above the other (sieve tower) and fixed in the sieve machine. The mesh sizes of sieves in the sieve tower increase in size from the bottom to the top of the tower. Analytical sieves (DIN ISO 3310-1) with dimensions of 200x50 mm are used.

An appropriate quantity of granules (80-120 g) is placed on the sieve with the largest mesh size (at the top of the sieve tower) and the granules are sieved with an amplitude of 1 mm for 2 minutes.

By weighing the product on every single sieve, a particle size distribution is calculated with the Retsch software ("EasySieve").

### Measurement of the Width/Length Ratio of the Piroctone Olamine Crystals

A small amount of sieved crystals (see above) is spread on a Petri dish with a spatula.

Under a microscope, a position is sought in which isolated crystals are clearly visible. The microscope used was a Keyence VHX 2000 series digital micoscope with a VH-Z20W zoom lens, using a VHX-S90BE free angle observation system. The microscope does not form part of the invention and a skilled person could select suitable alternative microscopes.

The limits are set for the depth of field, and an image in the appropriate magnification with the depth of field function of the microscope is made.

Pictures are taken at the following magnifications x50, x100, x150, x200 in order to obtain an overall impression of the bulk crystals.

A position on the Petri dish is needed in which an area of 3x3 images can be made with as many individual crystals as possible.

A 3x3 merged image (that is nine images, merged into one) is created at a magnification of x200.

The image is divided into 4 parts using a reticle scale. In each quarter 5 representative crystals are selected (20 crystals in total). For each of these 20 crystals, the diameter (D) and length (L) and the D/L ratio are determined. The average D/L ratio for all the crystals is then calculated, which is the sum of the measured D/L divided by the number of crystals (ΣD/L)/20).

### Measurement of the Width/Length Ratio of the Piroctone Olamine Granules

A small amount of sieved granules (see above) is spread on a Petri dish with a spatula.

Under a microscope, a position is sought in which isolated granules are clearly visible. The microscope used was a Keyence VHX 2000 series digital micoscope with a VH-Z20W zoom lens, using a VHX-S90BE free angle observation system. The microscope does not form part of the invention and a skilled person could select suitable alternative microscopes.

The limits are set for the depth of field, and an image in the appropriate magnification with the depth of field function of the microscope is made.

A position on the Petri dish is needed in which an area of 3x3 images can be made with as many individual granules as possible.

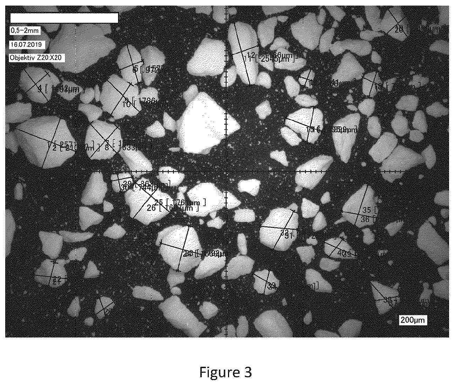

A 3x3 merged image (that is, nine images merged into one) is created at a magnification of x20. Figure 3 illustrates such an image.

The image is divided into 4 parts using a reticle scale. In each quarter 5 representative granules are selected (20 granules in total). For each of these 20 granules, the diameter (D) and length (L) and the D/L ratio are determined. The average D/L ratio for all the granules is then calculated, which is the sum of the measured D/L divided by the number of granules (ΣD/L)/20).

### Flowability Measurement

Clumping of the crystals or granules may be regarded as a low degree of flowability. In order to obtain an objective measurement of clumping, therefore, the crystals' / granules' flowability may be measured. The skilled person would be aware of other ways to characterize clumping.

The flowability of a bulk solid may be characterized by its unconfined yield strength, σ_{c}, in dependence on consolidation stress, σ₁, and storage period, t. Usually the ratio ff_{c} of consolidation stress, σ₁, to unconfined yield strength, σ_{c}, is used to characterize flowability numerically: ${ff}_{c} = {σ}_{1} / {σ}_{c}$

The larger ff_{c} is, i.e., the smaller the ratio of the unconfined yield strength, σ_{c}, to the consolidation stress, σ₁, the better a bulk solid flows. Flow behavior is defined as follows:
ff_{c} of less than 1, not flowing
ff_{c} from 1 to less than 2, very cohesive
ff_{c} from 2 to less than 4, cohesive
ff_{c} from 4 to less than 10, easy flowing
ff_{c} of greater than 10, free flowing

The parameter ff_{c} may be generated using a ring sheer test in the fashion described by Schulze, D (2009) "Pulver und Schuttguter", 2nd Edition, Springer, Berlin. This method does not form part of the present invention and is merely referred to as one way to characterize flowability in order to demonstrate the effect of the more flowable nature of the granules according to the invention versus piroctone olamine crystals. The skilled person would be aware of other ways to characterize flowability.

### Example 1

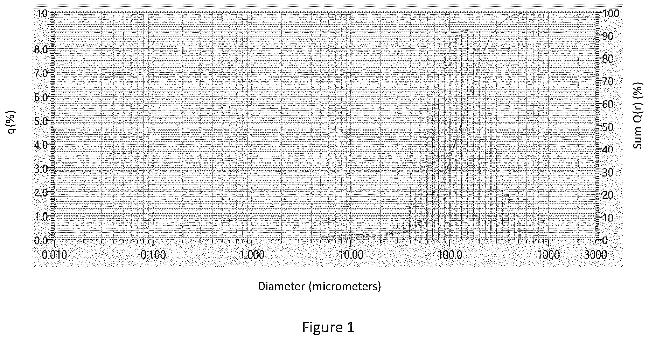

The starting material comprised 65% piroctone olamine crystals having the particle size distribution characteristics given in Table 1 and 35% of recycled fines (granules with d₅₀ of 0-0.5mm). The recycled fines consist of 100% compacted piroctone olamine from the previous run. Figure 1 illustrates the volumetric distribution density of particles, q (left hand y-axis) and the volumetric cumulative distribution Q(r) (right hand y-axis) versus diameter (x-axis) of crystals of piroctone olamine starting material.

| **Table 1** | |
|---|---|
| **d₁₀** | 0.06 mm |
| **dso** | 0.13 mm |
| **d₉₀** | 0.27 mm |
| **Average D/L** | 0.29 |

The devices used were as follows:
Compression Step a) was performed with a Hosokawa-Alpine "Pharmapaktor L200/50P" compactor having a concave plain roller diameter of 200 mm and width of 50 mm and a maximal compression force of 150 kN.

Milling Step b) was performed using with a Hosokawa-Alpine "FlakeCrusher FC200" sieve mill having a rotor diameter of 150 mm and a rotor length of 200 mm and a sieve mesh size of 2 mm.

Separating Step c) was performed using an Allgaier Vibrating Tumbler Screening Machine VTS 800 comprising a sieve of 800 mm diameter with a 0.50 mm mesh size.

Important parameters of the process in this example are given in Table 2. Piroctone olamine granules having a target particle size range is referred to below as "final product".

| **Table 2** | | |
|---|---|---|
| Recycled fines from a previous run | %wt | 35 |
| Fresh piroctone olamine crystals having the properties given in Table 1 | %wt | 65 |
| Bulk density of the starting material | kg/m³ | 464 |
| Rollers | | 2 concave plain rollers |
| Roller diameter | Mm | 200 |
| Working width of roller | Mm | 50 |
| Roller gap | Mm | 5 |
| Geometry of the feeding screw | | 60/66/120 |
| Target compression force | kN | 50 |
| Specific compression force | N/mm² | 5 |
| Roller rpm | Min⁻¹ | 12 |
| Feeding screw rpm | Min⁻¹ | 34 |
| Product temperature | °C | 38 |
| Compactor throughput | kg/h | 101 |
| Sieve mill FC200 throughput | kg/h | 250 |
| Compactate density | kg/m³ | 1030 |
| Compactate thickness | Mm | 5 |
| Final product bulk density | kg/m³ | 528 |
| Final product yield | %wt | 65% |
| Fines density (d₅₀ of 0-0.5mm) | kg/m³ | 460 |

The final product was analysed and had the properties given in Table 3. The image in Figure 3 was used to determine the average D/L value in the fashion described above.

| **Table 3** | |
|---|---|
| **d₁₀** | 0.5 mm |
| **d₅₀** | 1.1 mm |
| **d₉₀** | 1.8 mm |
| **Average D/L** | 0.84 |

For completeness, if the above process is performed in exactly the same way, but without recirculation of fines, then the final product bulk density is found to be 499 kg/m³, which is below the value of 528 kg/m³ measured (see Table 2) when fines are recirculated. A value of 499 kg/m³ is an acceptable final product bulk density, but the higher densities achieved via recirculation are preferred, because of the improved yield, as explained above.

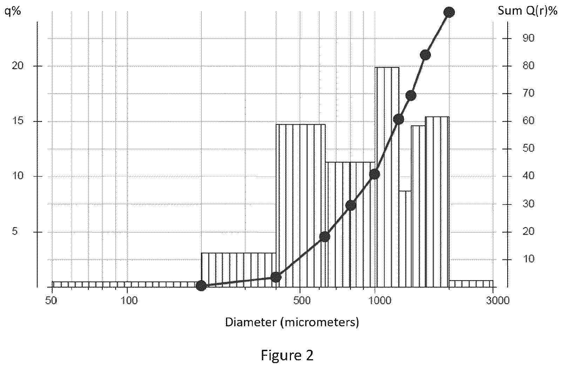

Figure 2 illustrates the volumetric distribution density of particles, q (left hand y-axis) and the volumetric cumulative distribution Q(r) (right hand y-axis) versus diameter (x-axis) of granules of piroctone olamine final product.

A flowability comparison between the starting material and the final product is provided in Table 4. In all cases, storage was at 35 degrees Celsius and 0% relative humidity under consolidation via application of a 2 kPa vertical pressure.

| **Table 4** | | |
|---|---|---|
| | **Flowability Factor, ff_{c}, measured after 20 hours** | **Flowability Factor, ff_{c}, measured after 168 hours** |
| **Starting material (piroctone olamine crystals having the properties in Table 1)** | 2.1, cohesive | N/A |
| **Final product (piroctone olamine granules having the properties in Table 3)** | 19.9, free flowing | 11.4, free flowing |

The results demonstrate that, under identical storage and consolidation conditions, piroctone olamine granules having the target particle size are significantly more flowable and therefore less liable to clumping than the non-recrystallized product.

### Examples of cosmetic compositions

### Example 2

### Cosmetic compositions (ingredients are given in wt%)

| **Example** | **2a** | **2b** | **2c** | **2d** | **2e** |
|---|---|---|---|---|---|
| Composition | Liquid soap | Body wash | Wet wipe lotion | Shampoo | Shampoo |
| Piroctone olamine granules | 0.5 | 0.5 | 0.5 | 0.5 | 0.5 |
| SLES | 10 | - | - | - | 9 |
| Cocamidopropyl betaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | - | - | - |
| Cocoyl Methyl Glucamide | - | 3 | 2 | - | - |
| EGDS | - | 0.5 | - | - | 0.7 |
| PEG-7 Glyceryl Cocoate | - | - | 1 | - | - |
| Cocoyl Glucoside | - | - | - | 5 | - |
| Lauryl Glucoside | - | - | - | 5 | - |
| Acrylates Copolymer | - | - | - | 2 | - |
| Glycerin | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| N-fatty acyl-N-methyl cyclic glucamide | 1.5 | 2 | 1.0 | 1.5 | 2 |
| NaCl | 1.5 | 1.0 | 0.5 | 0.3 | 1.5 |
| Water | QSP | QSP | QSP | QSP | QSP |
| Total | 100 | 100 | 100 | 100 | 100 |

### Example 3

### Cosmetic compositions (ingredients are given in wt%)

| **Example** | **3a** | **3b** | **3c** | **3d** |
|---|---|---|---|---|
| Composition | Body Lotion | Intensive hand and body lotion | Anti-ageing night cream | Baby cream |
| Piroctone olamine granules | 0.2 | 0.2 | 0.2 | 0.1 |
| Ethyhexyl Stearate | 7 | - | - | - |
| Decyl Oleate | 5 | - | - | - |
| Plantasens® Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex® AVC from Clariant (Ammonium Acryloyldimethyltaurate / VP Copolymer) | 0.5 | 1 | - | - |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens® Refined Organic Babassu Butter from Clariant (Orbignya Oleifera Seed Oil) | - | 2 | - | - |
| Squalane | - | 2 | 12 | - |
| Macadamia Ternifolia Seed Oil (and) Crambe Abyssinica Seed Oil (and) Orbignya Oleifera Seed Oil | - | 2 | - | - |
| Silcare® Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning® 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina® GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex® Velvet from Clariant (Polyacrylate Crosspolymer-11) | - | - | 0.3 | - |
| Hostacerin® SFO from Clariant (Sunflower Seed Oil Sorbitol Esters) | - | - | - | 3.5 |
| Paracera® M (Cera Microcristallina) | - | - | - | 2 |
| Beeswax | - | - | - | 1 |
| Magnesium Stearate | - | - | - | 1 |
| Talc | - | - | - | 10 |
| Zinc oxide | - | - | - | 10 |
| Allantoin (Clariant) | - | - | - | 0.3 |
| Extrapon Hamamelis | - | - | - | 2 |
| D-Panthenol | - | - | - | 2 |
| N-fatty acyl-N-methyl cyclic glucamide | 1.5 | 2 | 1.0 | 1.5 |
| Water | QSP | QSP | QSP | QSP |
| Total | 100 | 100 | 100 | 100 |

### Example 4

### Cosmetic compositions (ingredients are given in wt%)

| **Example** | **4a** | **4b** | **4c** | **4d** | **4e** | **4f** |
|---|---|---|---|---|---|---|
| Composition | Soap formulation | Soap bar | Body wash | Shampoo | Wash cream | Body wash |
| Piroctone olamine granules | 0.5 | 1.0 | 0.5 | 0.5 | 0.5 | 0.5 |
| Sodium Lauryl Sulfate | - | - | 10 | - | - | - |
| Ammonium Lauryl Sulfate | - | - | - | 12 | - | - |
| Sodium Lauryl Ether Sulfate (SLES) | 15 | - | 2 | - | - | - |
| Cocamidopropyl betaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerin | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| N-fatty acyl-N-methyl cyclic glucamide | 1.5 | 2 | 1.0 | 1.5 | 1 | 0.8 |
| NaCl | 1.5 | - | 0.5 | 0.3 | - | 2.5 |
| Water | QSP | QSP | QSP | QSP | QSP | QSP |
| Total | 100 | 100 | 100 | 100 | 100 | 100 |

### Example 5

### Hair conditioner compositions

| **Example** | **5a** | **5b** | **5c** | **5d** | **5e** | **5f** |
|---|---|---|---|---|---|---|
| Ingredient | wt.-% | wt.-% | wt.-% | wt.-% | wt.-% | wt.-% |
| Piroctone olamine granules | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 2.0 | - | - | 1.0 | 1.0 | - |
| BTAC | - | 2.0 | - | 1.0 | - | 1.0 |
| BTMS | - | - | 2.0 | - | 1.0 | 1.0 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance® Life: Clariant, INCI = Polyquaternium-116 und Butylene Glycol Genadvance® Repair: Clariant, INCI = Quaternium-98 Genadvance® Hydra: Clariant, INCI = Lauryl/Myristyl Polyricinoleate & Glycerin | | | | | | |

### Example 6

### Hair conditioner compositions

| **Example** | **6a** | **6b** | **6c** | **6d** | **6e** | **6f** |
|---|---|---|---|---|---|---|
| Ingredient | wt.-% | wt.-% | wt.-% | wt.-% | wt.-% | wt.-% |
| Piroctone olamine granules | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 0.5 | 0.5 | 0.5 | - | - | - |
| BTAC | - | - | - | 0.5 | 0.5 | 0.5 |
| Genadvance® Life | 1.5 | - | - | 1.5 | - | - |
| Genadvance® Repair | - | 1.5 | - | - | 1.5 | - |
| Genadvance® Hydra | - | - | 1.5 | - | - | 1.5 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance® Life: Clariant, INCI = Polyquaternium-116 und Butylene Glycol Genadvance® Repair: Clariant, INCI = Quaternium-98 Genadvance® Hydra: Clariant, INCI = Lauryl/Myristyl Polyricinoleate & Glycerin | | | | | | |

### Example 7

### Hair conditioner compositions

| **Example** | **7a** | **7b** | **7c** | **7d** | **7e** | **7f** |
|---|---|---|---|---|---|---|
| Ingredient | wt.-% | wt.-% | wt.-% | wt.-% | wt.-% | wt.-% |
| Piroctone olamine granules | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 0.2 | 0.2 | 0.2 | - | - | - |
| BTAC | - | - | - | 0.2 | 0.2 | 0.2 |
| Genadvance® Life | 1.8 | - | - | 1.8 | - | - |
| Genadvance® Repair | - | 1.8 | - | - | 1.8 | - |
| Genadvance® Hydra | - | - | 1.8 | - | - | 1.8 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance® Life: Clariant, INCI = Polyquaternium-116 und Butylene Glycol Genadvance® Repair: Clariant, INCI = Quaternium-98 Genadvance® Hydra: Clariant, INCI = Lauryl/Myristyl Polyricinoleate & Glycerin | | | | | | |

### Example 8

### Shampoo compositions

| **Example** | **8a** | **8b** | **8c** | **8d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 8 | 8 | 8 | 8 |
| sodium lauryl sulfate | 8 | 8 | 8 | 8 |
| cocamide MIPA | 4 | 4 | 4 | 4 |
| glycerin | 3.5 | 3.5 | 3.5 | 3.5 |
| glycol distearate | 3 | 3 | 3 | 3 |
| sodium chloride | 2.5 | 2.5 | 2.5 | 2.5 |
| dimethicone | 2 | 2 | 2 | 2 |
| PPG-5-ceteth-20 | 1.5 | 1.5 | 1.5 | 1.5 |
| Hair conditioning agent | 1 | 1 | 1 | 1 |
| carbomer | 0.4 | 0.4 | 0.4 | 0.4 |
| niacinamide | 0.2 | 0.2 | 0.2 | 0.2 |
| pyridoxine HCI | 0.2 | 0.2 | 0.2 | 0.2 |
| guar hydroxypropyltrimonium chloride | 0.2 | 0.2 | 0.2 | 0.2 |
| hydrolyzed soy protein | 0.2 | 0.2 | 0.2 | 0.2 |
| methyl cocoate | 0.15 | 0.15 | 0.15 | 0.15 |
| sodium cocoate | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 9

### Shampoo compositions

| **Example** | **9a** | **9b** | **9c** | **9d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 14 | 14 | 14 | 14 |
| coco-betaine | 4 | 4 | 4 | 4 |
| glycerin | 3 | 3 | 3 | 3 |
| glycol distearate | 2.5 | 2.5 | 2.5 | 2.5 |
| dimethicone | 2 | 2 | 2 | 2 |
| Hair conditioning agent | 1.5 | 1.5 | 1.5 | 1.5 |
| niacinamide | 0.5 | 0.5 | 0.5 | 0.5 |
| coconut oil | 0.5 | 0.5 | 0.5 | 0.5 |
| sodium chloride | 0.5 | 0.5 | 0.5 | 0.5 |
| olive fruit oil | 0.4 | 0.4 | 0.4 | 0.4 |
| PPG-5-ceteth-20 | 0.3 | 0.3 | 0.3 | 0.3 |
| trideceth-6 | 0.25 | 0.25 | 0.25 | 0.25 |
| polyquaternium-6 | 0.25 | 0.25 | 0.25 | 0.25 |
| amodimethicone | 0.2 | 0.2 | 0.2 | 0.2 |
| carbomer | 0.2 | 0.2 | 0.2 | 0.2 |
| cetrimonium chloride | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 10

### Shampoo compositions

| **Example** | **10a** | **10b** | **10c** | **10d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| ammonium lauryl sulfate | 16 | 16 | 16 | 16 |
| cocoamidopropyl betaine | 5 | 5 | 5 | 5 |
| sodium chloride | 2.5 | 2.5 | 2.5 | 2.5 |
| niacinamide | 2 | 2 | 2 | 2 |
| Hair conditioning agent | 1 | 1 | 1 | 1 |
| hydroxypropyl guar hydroxypropyltrimonium chloride | 0.8 | 0.8 | 0.8 | 0.8 |
| propylene glycol | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 11

### Shampoo compositions

| **Example** | **11a** | **11b** | **11c** | **11d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 16 | 16 | 16 | 16 |
| glycol distearate | 4 | 4 | 4 | 4 |
| coco-betaine | 2.5 | 2.5 | 2.5 | 2.5 |
| glycerin | 2 | 2 | 2 | 2 |
| sodium chloride | 1.5 | 1.5 | 1.5 | 1.5 |
| dimethicone | 1.5 | 1.5 | 1.5 | 1.5 |
| Hair conditioning agent | 1.5 | 1.5 | 1.5 | 1.5 |
| niacinamide | 1 | 1 | 1 | 1 |
| guar hydroxypropyltrimonium chloride | 0.8 | 0.8 | 0.8 | 0.8 |
| cocamide MIPA | 0.5 | 0.5 | 0.5 | 0.5 |
| PPG-5-ceteth-20 | 0.2 | 0.2 | 0.2 | 0.2 |
| fumaric acid | 0.15 | 0.15 | 0.15 | 0.15 |
| carbomer | 0.15 | 0.15 | 0.15 | 0.15 |
| pyridoxine HCI | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 12

### Shampoo compositions

| **Example** | **12a** | **12b** | **12c** | **12d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 16 | 16 | 16 | 16 |
| coco-betaine | 4 | 4 | 4 | 4 |
| niacinamide | 2 | 2 | 2 | 2 |
| Ribes nigrum oil | 1.5 | 1.5 | 1.5 | 1.5 |
| cocamide MIPA | 1.5 | 1.5 | 1.5 | 1.5 |
| sodium chloride | 1.5 | 1.5 | 1.5 | 1.5 |
| Hair conditioning agent | 1.2 | 1.2 | 1.2 | 1.2 |
| sodium cocoate | 0.8 | 0.8 | 0.8 | 0.8 |
| cocamide MIPA | 0.5 | 0.5 | 0.5 | 0.5 |
| Olea europaea oil (olive) fruit oil | 0.3 | 0.3 | 0.3 | 0.3 |
| PEG-55 propylene glycol oleate | 0.3 | 0.3 | 0.3 | 0.3 |
| PEG-60 hydrogenated castor oil | 0.3 | 0.3 | 0.3 | 0.3 |
| polyquaternium-7 | 0.2 | 0.2 | 0.2 | 0.2 |
| amodimethicone | 0.15 | 0.15 | 0.15 | 0.15 |
| propylene glycol | 0.1 | 0.1 | 0.1 | 0.1 |
| Butyrospermum parkii butter | 0.1 | 0.1 | 0.1 | 0.1 |
| laureth-5 carboxylic acid | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 13

### Shampoo compositions

| **Example** | **13a** | **13b** | **13c** | **13d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 10 | 10 | 10 | 10 |
| disodium cocamphodiacetate | 5 | 5 | 5 | 5 |
| glycerin | 3 | 3 | 3 | 3 |
| glycol distearate | 2 | 2 | 2 | 2 |
| sodium chloride | 1.5 | 1.5 | 1.5 | 1.5 |
| Hair conditioning agent | 1.5 | 1.5 | 1.5 | 1.5 |
| cocamide MEA | 1 | 1 | 1 | 1 |
| polyquaternium-10 | 1 | 1 | 1 | 1 |
| carbomer | 0.3 | 0.3 | 0.3 | 0.3 |
| citric acid | As needed, pH 4.5 - 6.5 | | | |
| sodium hydroxide | As needed, pH 4.5 - 6.5 | | | |
| Colorant | As needed | | | |

### Example 14

### Shampoo compositions

| **Example** | **14a** | **14b** | **14c** | **14d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 12 | 12 | 12 | 12 |
| coco-betaine | 3 | 3 | 3 | 3 |
| cocamide MIPA | 2 | 2 | 2 | 2 |
| sodium chloride | 1.8 | 1.8 | 1.8 | 1.8 |
| Olea europaea (olive) fruit oil | 0.5 | 0.5 | 0.5 | 0.5 |
| Hair conditioning agent | 0.5 | 0.5 | 0.5 | 0.5 |
| PPG-5-ceteth-20 | 0.4 | 0.4 | 0.4 | 0.4 |
| PEG-55 propylene glycol oleate | 0.4 | 0.4 | 0.4 | 0.4 |
| PEG-60 hydrogenated castor oil | 0.25 | 0.25 | 0.25 | 0.25 |
| polyquaternium-10 | 0.25 | 0.25 | 0.25 | 0.25 |
| amodimethicone | 0.15 | 0.15 | 0.15 | 0.15 |
| propylene glycol | 0.15 | 0.15 | 0.15 | 0.15 |
| laureth-5 | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 15

### Shampoo compositions

| **Example** | **15a** | **15b** | **15c** | **15d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| ammonium lauryl sulfate | 15 | 15 | 15 | 15 |
| cocamidopropyl betaine | 3 | 3 | 3 | 3 |
| sodium chloride | 2 | 2 | 2 | 2 |
| propylene glycol | 1.8 | 1.8 | 1.8 | 1.8 |
| hydroxypropyl guar hydroxypropyltrimonium chloride | 1.5 | 1.5 | 1.5 | 1.5 |
| Hair conditioning agent | 1 | 1 | 1 | 1 |
| citric acid | As needed, pH 4.5 - 6.5 | | | |
| sodium hydroxide | As needed, pH 4.5 - 6.5 | | | |
| Colorant | As needed | | | |

### Example 16

### Shampoo compositions

| **Example** | **16a** | **16b** | **16c** | **16d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium cocoyl isethionate | 4 | 4 | 4 | 4 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.5 | 2.5 | 2.5 | 2.5 |
| glycol distearate | 2 | 2 | 2 | 2 |
| glycereth-26 | 1.8 | 1.8 | 1.8 | 1.8 |
| decyl glucoside | 1.5 | 1.5 | 1.5 | 1.5 |
| hydrogenated coconut acid | 1.4 | 1.4 | 1.4 | 1.4 |
| PPG-5-ceteth-20 | 1.3 | 1.3 | 1.3 | 1.3 |
| divinyldimethicone/dimethicone copolymer | 1.2 | 1.2 | 1.2 | 1.2 |
| sodium chloride | 1.1 | 1.1 | 1.1 | 1.1 |
| amodimethicone | 1 | 1 | 1 | 1 |
| Hair conditioning agent | 1 | 1 | 1 | 1 |
| polyquaternium-7 | 1 | 1 | 1 | 1 |
| polyquaternium-10 | 0.7 | 0.7 | 0.7 | 0.7 |
| sodium isethionate | 0.5 | 0.5 | 0.5 | 0.5 |
| PEG-55 propylene glycol oleate | 0.4 | 0.4 | 0.4 | 0.4 |
| propylene glycol | 0.4 | 0.4 | 0.4 | 0.4 |
| carbomer | 0.4 | 0.4 | 0.4 | 0.4 |
| C11-15 pareth-7 | 0.3 | 0.3 | 0.3 | 0.3 |
| glycerin | 0.3 | 0.3 | 0.3 | 0.3 |
| trideceth-12 | 0.25 | 0.25 | 0.25 | 0.25 |
| laureth-9 | 0.2 | 0.2 | 0.2 | 0.2 |
| hydroxypropyltrimonium hydrolyzed wheat protein | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 17

### Shampoo compositions

| **Example** | **17a** | **17b** | **17c** | **17d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium methyl cocoyl taurate | 5 | 5 | 5 | 5 |
| laureth-5 carboxylic acid | 4 | 4 | 4 | 4 |
| sodium chloride | 2 | 2 | 2 | 2 |
| cocamidopropyl betaine | 2 | 2 | 2 | 2 |
| glycerin | 1.8 | 1.8 | 1.8 | 1.8 |
| glycol distearate | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5 ceteth-20 | 0.5 | 0.5 | 0.5 | 0.5 |
| sodium lauroyl glutamate | 0.4 | 0.4 | 0.4 | 0.4 |
| propylene glycol | 0.2 | 0.2 | 0.2 | 0.2 |
| PEG-55 propylene glycol oleate | 0.15 | 0.15 | 0.15 | 0.15 |
| Argania spinosa kernel oil | 0.1 | 0.1 | 0.1 | 0.1 |
| citric acid, lactic acid | As needed, pH 4.5 - 6.5 | | | |
| sodium hydroxide | As needed, pH 4.5 - 6.5 | | | |
| Colorant | As needed | | | |

### Example 18

### Shampoo compositions

| **Example** | **18a** | **18b** | **18c** | **18d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 10 | 10 | 10 | 10 |
| disodium cocoamphodiacetate | 4 | 4 | 4 | 4 |
| glycol distearate | 2 | 2 | 2 | 2 |
| sodium chloride | 1.5 | 1.5 | 1.5 | 1.5 |
| Hair conditioning agent | 1.3 | 1.3 | 1.3 | 1.3 |
| carbomer | 0.4 | 0.4 | 0.4 | 0.4 |
| diaminopyrimidine oxide | 0.3 | 0.3 | 0.3 | 0.3 |
| disodium ricinoleamido MEA sulfosuccinate | 0.3 | 0.3 | 0.3 | 0.3 |
| propylene glycol | 0.2 | 0.2 | 0.2 | 0.2 |
| panthenol | 0.1 | 0.1 | 0.1 | 0.1 |
| polyquaternium-10 | 0.1 | 0.1 | 0.1 | 0.1 |
| citric acid | As needed, pH 4.5 - 6.5 | | | |
| sodium hydroxide, ammonium hydroxide | As needed, pH 4.5 - 6.5 | | | |
| Colorant | As needed | | | |

### Example 19

### Shampoo compositions

| **Example** | **19a** | **19b** | **19c** | **19d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium cocoyl isethionate | 4.5 | 4.5 | 4.5 | 4.5 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.3 | 2.3 | 2.3 | 2.3 |
| glycol distearate | 2 | 2 | 2 | 2 |
| glycereth-26 | 1.8 | 1.8 | 1.8 | 1.8 |
| decyl glucoside | 1.7 | 1.7 | 1.7 | 1.7 |
| hydrogenated coconut acid | 1.5 | 1.5 | 1.5 | 1.5 |
| PPG-5-ceteth-20 | 1.5 | 1.5 | 1.5 | 1.5 |
| PEG-55 propylene glycol oleate | 1.5 | 1.5 | 1.5 | 1.5 |
| propylene glycol | 1.5 | 1.5 | 1.5 | 1.5 |
| sodium chloride | 1.5 | 1.5 | 1.5 | 1.5 |
| divinyldimethicone/dimethicone copolymer | 1 | 1 | 1 | 1 |
| polyquaternium-7 | 1 | 1 | 1 | 1 |
| amodimethicone | 1 | 1 | 1 | 1 |
| Hair conditioning agent | 1 | 1 | 1 | 1 |
| polyquaternium-10 | 0.8 | 0.8 | 0.8 | 0.8 |
| sodium isethionate | 0.6 | 0.6 | 0.6 | 0.6 |
| carbomer | 0.6 | 0.6 | 0.6 | 0.6 |
| C11-15 pareth-7 | 0.4 | 0.4 | 0.4 | 0.4 |
| glycerin | 0.3 | 0.3 | 0.3 | 0.3 |
| laureth-9 | 0.2 | 0.2 | 0.2 | 0.2 |
| citric acid | As needed, pH 4.5 - 6.5 | | | |
| sodium hydroxide | As needed, pH 4.5 - 6.5 | | | |
| Colorant | As needed | | | |

### Example 20

### Shampoo compositions

| **Example** | **20a** | **20b** | **20c** | **20d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| cocamidopropyl betaine | 5 | 5 | 5 | 5 |
| sodium lauryl sulfoacetate | 4 | 4 | 4 | 4 |
| disodium laureth sulfosuccinate | 3.5 | 3.5 | 3.5 | 3.5 |
| sodium lauroyl sarcosinate | 3 | 3 | 3 | 3 |
| glycol distearate | 2 | 2 | 2 | 2 |
| sodium chloride | 1.7 | 1.7 | 1.7 | 1.7 |
| decyl glucoside | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5-ceteth-20 | 1 | 1 | 1 | 1 |
| coco-betaine | 0.8 | 0.8 | 0.8 | 0.8 |
| PEG-55 propylene glycol oleate | 0.8 | 0.8 | 0.8 | 0.8 |
| propylene glycol | 0.5 | 0.5 | 0.5 | 0.5 |
| carbomer | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 21

### Shampoo compositions

| **Example** | **21a** | **21b** | **21c** | **21d** |
|---|---|---|---|---|
| Ingredient | wt% | wt% | wt% | wt% |
| Aqua | To 100 | To 100 | To 100 | To 100 |
| Piroctone olamine granules | 0.1 | - | - | - |
| Piroctone olamine granules | - | 0.3 | - | - |
| Piroctone olamine granules | - | - | 0.7 | - |
| Piroctone olamine granules | - | - | - | 1.0 |
| sodium laureth sulfate | 15 | 15 | 15 | 15 |
| coco-betaine | 3 | 3 | 3 | 3 |
| glycol distearate | 2.5 | 2.5 | 2.5 | 2.5 |
| sodium chloride | 2 | 2 | 2 | 2 |
| PPG-5-ceteth-20 | 1.8 | 1.8 | 1.8 | 1.8 |
| Hair conditioning agent | 1.5 | 1.5 | 1.5 | 1.5 |
| octyldodecanol | 1 | 1 | 1 | 1 |
| carbomer | 1 | 1 | 1 | 1 |
| guar hydroxypropyltrimonium chloride | 1 | 1 | 1 | 1 |
| sodium hyaluronate | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

## Claims

1. Use of piroctone olamine granules for the manufacture of a cosmetic composition.

2. Use according to claim 1, wherein the piroctone olamine granules have a d50 from 0.2 mm to 8 mm, preferably from 0.3 mm to 6 mm, more preferably from 0.4 mm to 2.5 mm, particularly preferably from 0.5 mm to 2 mm.

3. Use according to claim 1 or 2, wherein the piroctone olamine granules have an average D/L ratio of diameter (D) to length (L) from 0.6 to 1.0, preferably from 0.7 to 1.0, particularly preferably from 0.8 to 1.0.

4. Use according to any of claims 1 to 3, wherein the piroctone olamine granules have a d90 of less than or equal to 1.9 mm.

5. Use according to any of claims 1 to 4, wherein the piroctone olamine granules have a d10 of greater than or equal to 0.4 mm.

6. Use according to any of claims 1 to 5, wherein the piroctone olamine granules have a bulk density from 400 kg/m³ to 600 kg/m³, preferably from 450 kg/m³ to 550 kg/m³, more preferably from 470 kg/m³ to 530 kg/m³.

7. Use according to any of claims 1 to 6, wherein from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of the piroctone olamine granules, based on the total weight of the cosmetic composition, are used to manufacture the cosmetic composition.

8. Use according to any of claims 1 to 7, wherein the cosmetic composition is selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask, face cream, hand cream and body lotion.

9. Use according to any of claims 1 to 8, wherein the piroctone olamine granules are obtained by a process comprising
a) compressing piroctone olamine crystals to form a compactate;
b) milling the compactate to form piroctone olamine granules.

10. Use according to claim 9, wherein the process further comprises
c) separating the piroctone olamine granules according to their particle size.

11. Use according to claim 9 or 10, wherein the compression in a) is performed at a pressure from 25 bar to 200 bar, preferably from 30 bar to 50 bar.

12. Use according to any of claims 9 to 11, wherein the compression in a) is performed in the absence of any additives.

13. Use according to any of claims 9 to 12, wherein in b) milling takes place in a sieve mill, preferably a rotary sieve mill or an oscillating sieve mill.

14. Cosmetic composition comprising piroctone olamine granules as defined in any of claims 1 to 13.

15. Use of piroctone olamine granules as defined in any of claims 1 to 13 as an antidandruff agent or as a preservative.
